# EUROPEAN PATENT SPECIFICATION

(11) **EP 2 750 666 B1**
(45) Date of publication and mention of the grant of the patent: **04.10.2017**
(21) Application number: 12759055.2
(22) Date of filing: 30.08.2012
(51) Int. Cl.: A61K 9/20, A61K 9/16, A61K 31/196, A61K 31/549, A61K 31/138

(54) **MULTI-LAYERED RELEASE FORMULATION**
MEHRSCHICHTIGE FORMULIERUNGSFREISETZUNG
FORMULATION DE LIBÉRATION MULTICOUCHES

(30) Priority: 30.08.2011 EP 11179362
(43) Date of publication of application: 09.07.2014
(73) Proprietor: Universiteit Gent, 9000 Gent (BE)
(72) Inventor: REMON, Jean Paul, 9090 Melle (BE); VERVAET, Chris, 8870 Kachtem (BE)
(74) Representative: LC Patents
(86) International application number: PCT/EP2012/066848
(87) International publication number: WO 2013/030267

(56) References cited:
- WO-A1-02/051407
- WO-A1-2004/028503
- WO-A2-2005/051234
- US-A1- 2009 130 058
- HARDUNG H ET AL: "Combining HME & solubilization: Soluplus(R) - The solid solution", DRUG DELIVERY TECHNOLOGY, DRUG DELIVERY TECHNOLOGY, US, vol. 10, no. 3, 1 April 2010 (2010-04-01), pages 20-27, XP009155307, ISSN: 1537-2898
- DIERICKX L ET AL: "Co-extrusion as manufacturing technique for fixed-dose combination mini-matrices", EUROPEAN JOURNAL OF PHARMACEUTICS AND BIOPHARMACEUTICS, ELSEVIER SCIENCE PUBLISHERS B.V., AMSTERDAM, NL, vol. 81, no. 3, 4 April 2012 (2012-04-04), pages 683-689, XP028405177, ISSN: 0939-6411, DOI: 10.1016/J.EJPB.2012.03.018 [retrieved on 2012-04-04]

## Description

### FIELD OF THE INVENTION

The present invention in general relates to an oral pharmaceutical dosage form comprising a multi-layered release formulation formed by co-extrusion. Said formulation in particular comprises a core layer comprising at least one polymer selected from polycaprolactone, ethylcellulose, and/or combinations thereof; and a coat layer comprising at least one (co)polymer selected from the group consisting of: a combination of polyethylene oxide and polyethylene glycol (PEG); Basic Butylated Methacrylate (co)polymer; a (co)polymer of polyvinylcaprolactam, PEG and polyvinylacetate; and combinations thereof.

### BACKGROUND TO THE INVENTION

Conventional pharmaceutical dosage forms are often associated with undesired drug level oscillations. For many drugs, however, controlled release may be desired, making conventional dosage forms less suitable. Therefore, controlled and/or sustained-release delivery systems are continuously being developed for multiple types of drugs. In said novel delivery systems, preferably drug level oscillations should be minimized and more constant controlled drug levels over time should be achieved by the use of said controlled and/or sustained-delivery formulations. Even though various drug delivery systems are used for maximizing therapeutic index and reducing the side effects of the drug, oral route remains the preferred, promising and effective route for the administration of therapeutic agents. Low cost of therapy, ease of administration, flexibility in formulation and handling leads to higher level of patient compliance. Approximately 50% of the drug delivery systems available in the market are oral drug delivery systems. Design of oral delivery systems containing drugs however are challenging in view of the acidic environment of the stomach before entering the intestine, with increased pH, presence of bile salts and enzymes.

Ideally, controlled and/or sustained-release formulations should be capable of providing a therapeutically effective drug level which allows the practitioner to target the therapeutic window of drug efficacy, while controlling the drug levels. In addition, controlled and/or sustained-release formulations may also minimize the frequency of dosing, which has a positive impact on patient compliance.

For certain conditions, it may be advantageous to provide multi-layered release formulations capable of delivering therapeutics in a bi-phasic or multi-phasic controlled fashion, rather than a single phase extended release preparation. For example, in a first phase of drug release, an immediate release dose fraction reaches a therapeutic drug level quickly after administration, while in an extended second release phase, the drug is released over a prolonged period of time, maintaining the desired therapeutic level. Alternatively, multi-layered release formulations may be very suitable for releasing two or more types of drugs in a different release fashion, for example one in a quick release fashion, another over a prolonged period of time.

Currently available multi-layered drug release formulations are often pellets or particles comprising a core and a coat, wherein the coating is applied in a batch manufacturing process. However, batch process manufacturing has many drawbacks. Furthermore, quality is assessed through sampling during the process, and if quality standards are not met, the entire batch is rejected. It is therefore desirable to manufacture pharmaceutical multi-layered compositions by a continuous manufacturing method.

Continuous manufacturing of multi-layered drug formulations may for example include co-extrusion of a core and a coat layer. However, it is evident that in such case, the formulation should fulfill a number of requirements:
- the (co)polymers used in the core and coat should be mutually compatible,
- the (co)polymers used in both layers should have similar rheological properties, so that they can be co-extruded under the same conditions of pressure and temperature,
- during the co-extrusion process, the coat layer(s) should remain separate from the core layer, but still sufficiently adhere thereto,
- the used (co)polymers should have the desired drug release profile.

It was therefore an object of this invention to provide a co-extruded multi-layered pharmaceutical formulation that fulfills the criteria as specified above, having at least one immediate release coat layer and a prolonged controlled release core layer. Keeping the above criteria in mind, we have now found that polycaprolactone and/or ethylcellulose are very suitable core materials for obtaining a multi-layered pharmaceutical formulation prepared by co-extrusion.

The use of ethylcellulose in pharmaceutical compositions is known. For example US6787156 provides a pharmaceutical composition comprising a matrix, which is erodible in aqueous medium and a coating comprising at least one cellulose derivative such as ethylcellulose. Disclosed matrix compounds do not include polycaprolactone and only includes ethylcellulose matrices in combination with an ethylcellulose coating layer.

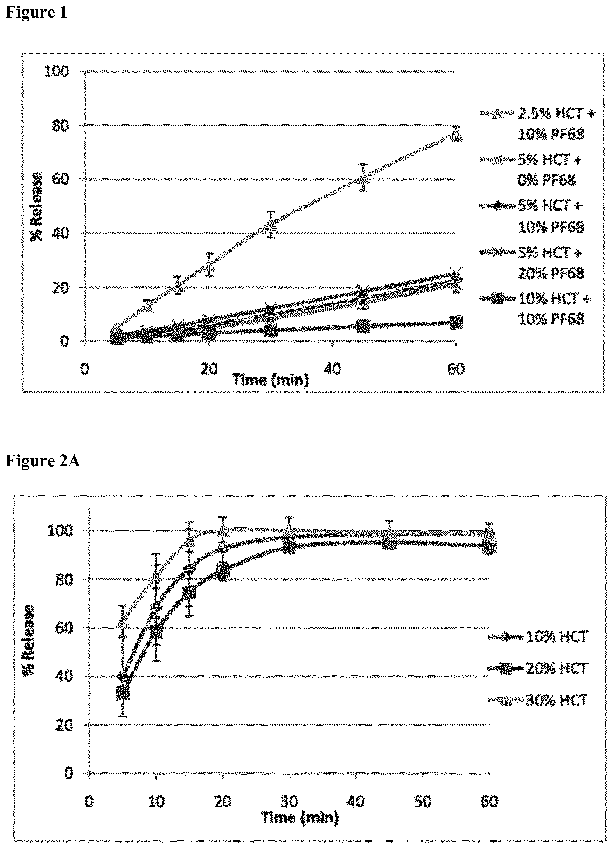

WO2004028503 discloses an open reservoir system consisting of an ethylcellulose pipe surrounding a drug containing core, said core consisting of a hydrophilic cellulose polymer. Furthermore, as evident from figures 1-4, the exemplified open reservoir systems do not provide the desired release profile, and are thus considered not to be suitable to provide a solution to the problems as defined above.

US20090130058 discloses a pharmaceutical melt-extruded composition comprising at least 25% wt of ethylcellulose, and an ethylene oxide homo-or copolymer. The exemplified compositions, however only contain a core, not a coat layer, and no indications are provided which types of materials could be useful for co-extrusion with a coat layer. In addition, the exemplified compositions, do not have the desired release profile compared to the compositions according to this invention.

WO2003094888 provides a method of making a drug delivery device by co-extruding a core and at least one outer polymeric skin. The exemplified core materials comprises FA (flucinolone acetonide) in admixture with poly(vinyl acetate), polycaprolactone, PEG or PLGA. No indications are provided that ethylcellulose could be used as core material in a biphasic or multi-phasic co-extruded formulation. In addition, no exemplified formulations are provided wherein the core layer comprises polycaprolactone. Furthermore, the exemplified release profiles are very different compared to the compositions according to this invention.

WO2005051234 discloses an open reservoir injectable drug delivery device comprising a drug containing polycaprolactone core optionally surrounded by a polymeric skin. Again the exemplified coatings and release profiles are very different compared to the compositions according to this invention.

WO2010032128 provides an extended release pharmaceutical dosage form comprising a melt-formed multi-particulate extended release matrix formulation comprising at least one poly(ε-caprolactone), optionally in combination with at least one polyethylene oxide. WO2010032128 does not provide multi-particulates comprising a coat, nor indications of what kind of materials could be used therefore.

### SUMMARY OF THE INVENTION

The current invention provides a pharmaceutical formulation useful for oral and controlled delivery of one or more active ingredients, especially in a bi-phasic or multi-phase manner.

The present invention relates to a solid pharmaceutical oral dosage form, comprising a co-extrusion formed multi-layered release formulation having:
- a core layer comprising at least one polymer selected from polycaprolactone, ethylcellulose, and/or combinations thereof, and when the polymer is ethylcellulose the core layer contains at least one plasticizer; and
- a coat layer comprising at least one (co)polymer selected from the group consisting of: a combination of polyethylene oxide and polyethylene glycol (PEG); Basic Butylated Methacrylate (co)polymer; a (co)polymer of polyvinylcaprolactam, PEG and polyvinylacetate; and combinations thereof; and
wherein at least one of said layers comprises an active ingredient.

In a specific embodiment, at least two of the layers of the multi-layered release formulation comprises an active ingredient. Even more specific, each of the layers of the multi-layered release formulation comprises an active ingredient.

The multi-layered release formulation according to this invention may further comprise one or more additional core and/or coat layers. The core layer and the coat layer(s) may comprise the same or a different active ingredient.

In a preferred embodiment, the solid pharmaceutical oral dosage form according to this invention comprises polycaprolactone, or ethylcellulose at an amount of between 20 and 99,9 weight-% of the core layer. The (co)polymer of the coat layer(s) is preferably present at an amount of between 10 to 99,9 weight-% of the coat layer.

In another preferred embodiment wherein the dosage form has a cylindrical shape, the core layer of the solid pharmaceutical dosage form according to this invention has a diameter in the range of 0.1 to 3 mm.

In yet another preferred embodiment wherein the dosage form has a cylindrical shape, the coat layer(s) of the solid pharmaceutical dosage form according to this invention, taken together have a thickness in the range of 0.1 to about 3 mm.

Both the core layer and the coat layer(s) of the solid pharmaceutical oral dosage form, may further comprise at least one plasticizer.

This invention further provides the solid pharmaceutical oral dosage form according to this invention for use as a medicament, in particular for the bi-phasic or multi-phasic release of one or more active ingredients.

Futhermore, the invention provides a solid pharmaceutical oral dosage form for use in the treatment of a disease by delivering one or more active ingredients to a subject in a bi-phasic or multi-phasic manner.

In a further aspect, the present invention provides a method for the bi-phasic or multi-phasic release of one or more active ingredients, said method comprising administering a solid pharmaceutical oral dosage form according to this invention.

In yet a further aspect, the present invention provides a method of preparing a multi-layered release formulation; said method comprising co-extruding:
- a core layer comprising at least one polymer selected from polycaprolactone, ethylcellulose, and/or combinations thereof, and when the polymer is ethylcellulose the core layer contains at least one plasticizer; and
- a coat layer comprising at least one (co)polymer selected from the group consisting of a combination of polyethylene oxide and polyethylene glycol; Basic Butylated Methacrylate (co)polymer; a (co)polymer of polyvinylcaprolactam, PEG and polyvinylacetate; and combinations thereof.

The current invention also provides a multi-layered release formulation obtainable by a process as defined herein.

### BRIEF DESCRIPTION OF THE DRAWINGS

**Figure 1****:** 60-min release profile of formulations comprising Soluplus® and different amounts of hydrochlorothiazide and Pluronic® F68.
**Figure 2****:** 60-min release profile of formulations comprising Eudragit® E PO and different amounts of hydrochlorothiazide (A) or 10% hydrochlorothiazide and varying amounts of triethylcitrate (B).
**Figure 3****:** 60-min release profile of formulations comprising Polyethylene oxide (molecular weight = 100 000 g/mol) and different amounts of hydrochlorothiazide (A). 60-min release profile of formulations comprising Polyethylene oxide (molecular weight = 100 000 g/mol) (PEO 100.000) and polyethylene glycol (MW = 4000 g/mol) (PEG 4000) at PEO 100.000/PEG 4000 (1/1) and different amounts of hydrochlorothiazide (B).
**Figure 4****:** 24-h release profile of formulations comprising Eudragit® RS PO and different amounts of metoprolol tartrate (A); or 10% dibutylsebacate and varying amounts of metoprolol tartrate (B); or 10% dibutylsebacate, 30 % metoprolol tartrate and varying sizes (C).
**Figure 5****:** 24-h release profile of formulations comprising Eudragit® NE 30D (freeze dried) and 30% metoprolol tartrate and extruded at different temperatures.
**Figure 6****:** 24-h release profile of formulations comprising Capa®6506 and varying amounts of metoprolol tartrate (A), or comprising 40% metoprolol tartrate and having varying sizes (B).
**Figure 7****:** 24-h release profile of formulations comprising Ethocel® STD 10 having a size of 2x2 mm and varying amounts of metoprolol tartrate (A), or having a size of 3x2mm and varying amounts of metoprolol tartrate (B).
**Figure 8****:** 24-h release profile of a formulation comprising a core layer comprising 45% metoprolol tartrate in polycaprolactone (A). 60 min release profile of a formulation comprising a coat layer comprising 10% hydrochlorothiazide in PEO 100.000/PEG 4000 (1/1) (B). 24-h cumulative release profile of a formulation consisting of a core layer comprising 45% metoprolol tartrate in polycaprolactone, (represented by grey squares) and a coat layer comprising 10% hydrochlorothiazide in PEO 100.000/PEG 4000 (1/1) (C) (represented by white diamonds).
**Figure 9****:** 24-h release profile of a formulation consisting of a core layer comprising 50% sodium diclofenac in polycaprolactone, and a coat layer comprising 22,5% sodium diclofenac in PEO 100.000/PEG 4000 (1/1).
**Figure 10****:** 24-h release profile of a formulation consisting of a core layer comprising 60% sodium diclofenac in ethylcellulose and 20% dibutyl sebacate, and a coat layer comprising 26% sodium diclofenac in Soluplus ® and 10% Pluronic ® F68.
**Figure 11****:** 24-h release profile of formulations consisting of a core layer comprising varying amounts of metoprolol tartate in ethylcellulose and 15% dibutyl sebacate, and a coat layer comprising hydrochlorothiazide in Eudragit ® E PO.
**Figure 12****:** 60-min release profile of hydrochlorothiazide from formulations consisting of a core layer comprising varying amounts of MPT in ethylcellulose and varying amounts of dibutyl sebacate and PEO 1M; and a coat layer comprising varying amounts of hydrochlorothiazide in 85% PEO 1M and 15% PEG 4000.
**Figure 13****:** 24-h release profile of metoprolol tartate from formulations consisting of a core layer comprising varying amounts of MPT in ethylcellulose and varying amounts of dibutyl sebacate and PEO 1M; and a coat layer comprising varying amounts of hydrochlorothiazide in 85% PEO 1M and 15% PEG 4000.
**Figure 14****:** Mean MPT (■) and HCT (▲) plasma concentration-time profiles (± SD, n = 6) after oral administration of 200 mg metoprolol tartrate and 25 mg hydrochlorothiazide to dogs: Zok-Zid® (2 tablets) (dotted line), experimental co-extruded mini-matrices with a core consisting of 45% (w/w) MPT and a coat consisting of 10% (w/w) HCT (full line).
**Figure 15****:** Comparison of AUC level of MPT (A) and HCT (B) after oral administration of experimental co-extruded and reference formulation to dogs.
**Figure 16****:** Mean MPT (full lines) and HCT (dotted lines) plasma concentration-time profiles (± SD, n = 6) after oral administration of 200 mg metoprolol tartrate and 25 mg hydrochlorothiazide to dogs in experimental co-extruded mini-matrices consisting of a core layer comprising varying amounts of MPT in ethylcellulose and varying amounts of dibutyl sebacate and PEO 1M; and a coat layer comprising varying amounts of hydrochlorothiazide in 85% PEO 1M and 15% PEG 4000

### DETAILED DESCRIPTION OF THE INVENTION

The current invention provides a pharmaceutical formulation useful for oral and controlled delivery of one or more active ingredients. The formulation is a multi-layered dosage form for delivering therapeutics in a bi-phasic (two phases) or multi-phasic (three, four or more phases) controlled fashion, rather than a single phase sustained release preparation. One or more active ingredients can be released in one or more initial phases wherein an immediate release dose fraction reaches a therapeutic drug level shortly after administration, while in an extended release phase, active ingredients can be released over a prolonged period of time, maintaining the desired therapeutic level. Alternatively, the multi-layered release formulation is capable of releasing two or more different types of drugs in a different release fashion, for example one or more in a quick release fashion and other(s) over a prolonged period of time.

The present invention relates to a solid pharmaceutical oral dosage form, comprising a co-extrusion formed multi-layered release formulation, comprising, consisting essentially of, or consisting of:
- at least one core layer comprising at least one polymer selected from polycaprolactone, ethylcellulose, and combinations thereof, and when the polymer is ethylcellulose the core layer contains at least one plasticizer; and
- at least one coat layer comprising at least one (co)polymer selected from the group consisting of a combination of polyethylene oxide and polyethylene glycol (PEG); Basic Butylated Methacrylate (co)polymer; a (co)polymer of polyvinylcaprolactam, PEG and polyvinylacetate; or combinations thereof; and
wherein at least one of said layers comprises an active ingredient, more in particular wherein at least two of said layers comprise an active ingredient.

In a specific embodiment of the multi-layered formulation, 3, 4, 5, or more of said layers comprises an active ingredient. Even more specific, each of the layers comprises an active ingredient.

A "solid" dosage form refers to a dosage form of definite shape and volume, not liquid or gaseous. The dosage form of the present invention may be multi-layered, meaning that it may exist of one or more, i.e. 2, 3, 4, 5 or more, layers or sheets.

"At least one ... layer" as used herein is meant to include 1, 2, 3, 4, 5 or more ... layers. In particular, the at least one core layer comprises at least one polymer selected from polycaprolactone, ethylcellulose, and combinations thereof. Furthermore, the at least one coat layer of the multi-layered formulation comprises at least one (co)polymer selected from the group consisting of a combination of polyethylene oxide and polyethylene glycol (PEG); Basic Butylated Methacrylate (co)polymer; a (co)polymer of polyvinylcaprolactam, PEG and polyvinylacetate; or combinations thereof.

The shape of the multi-layered formulation can be cylindrical, angular, square, a flat sheet or any other shape, but preferably is cylindrical. In said embodiment, the core or inner layer(s) is/are surrounded by (a) coat or outer layer(s). In particular, the coat layer at least partially surround the inner core layer, or as an alternative completely surrounds the inner core.

In case of a multi-layered sheet, the terms "core" and "coat" layer can be replaced by "immediate release" and "sustained release" layer.

As used herein, "controlled or sustained-release" refers to the release of an active ingredient from a pharmaceutical dosage form at a predetermined rate. Controlled or sustained release implies that the active ingredient is not released in an unpredictable fashion and that the majority of the active ingredient does not "burst" off of the dosage form upon contact with a biological environment. On the other hand, "immediate release" implies that the majority of the drug does burst off of the dosage form upon contact with a biological environment. For example, in case of an oral dosage form, the majority of the drug in the coat or immediate release layer will burst off upon contact with the acidic environment of the stomach.

It is an aim of the present invention to provide a multi-layered formulation for delivering one, two or more active ingredients in a bi-phasic or multi-phasic controlled fashion, rather than a single phase sustained release preparation. The formulations of the present invention offer the opportunity to modulate drug release either by loading the different layers with different amounts/types of drug or by incorporating the drug(s) in different matrices. Furthermore it allows the simultaneous administration of non-compatible drugs (formulated in separate layers) and to combine different drugs with different release profiles.

As already indicated herein before, it was an object of this invention to provide a solid pharmaceutical dosage form, that can be manufactured by co-extrusion, which comprises a controlled release core or inner layer and at least one immediate release coat or outer layer, preferably resulting in a release profile as further detailed in the examples that follow hereinafter.

Co-extruded dosage forms are matrix formulations into which the drug is homogeneously embedded. The dosage form of the present invention is thus specifically useful to deliver one active ingredient with at least two different release rates or to deliver at least two or more different active ingredients at different release rates.

In a particular embodiment of this invention, the solid pharmaceutical dosage forms as described herein provide a first burst off phase (immediate release) in which the majority, i.e. about 80, 85, 90, 95 or 100%, of the active ingredient(s) comprised in the immediate release or coat layer(s) of the formulation is released within the first hour after administration of the pharmaceutical dosage form, preferably within the first 50, 40 or 30 minutes, most preferably within the first 20 minutes. In the second release phase (sustained release), the majority i.e. about 80, 85, 90, 95 or 100% of the active ingredient(s) comprised in the controlled release or core layer is gradually released within the first 96 hours after administration of the pharmaceutical dosage form, preferably within the first 72, or 48 hours, most preferably within the first 24 hours.

The "sustained release" layer in the context of this invention preferably exhibits a release profile such that after 1 hour not more than 20% of the active ingredient is released. The release profile can be determined in vitro as described in the present examples, e.g. in USP hydrochloric acid (pH 1) at 37°C in an USP Apparatus 1.

As used herein, polycaprolactone is represented by the following general formula, preferably having an average molecular weight of about 40.000 - 60.000 g/mol, in particular about 50.000 g/mol:

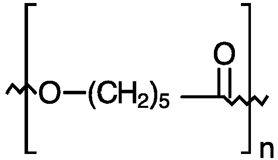

Ethylcellulose as used herein represents a cellulose derivative in which some of the hydroxyl groups on the repeating glucose units are converted into ethyl ether groups. The number of ethyl groups can vary depending on the manufacture. Ethylcellulose is represented by the following general formula, wherein R is selected from -H or -ethyl.

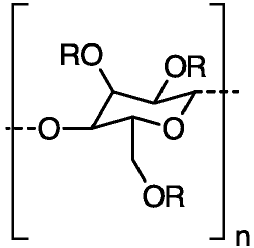

Ethylcellulose as used in the present invention when prepared as a 5% solution in toluene/ethanol (80/20) preferably has a viscosity of about 9-11 mPa.s. It further preferably has an ethoxyl content of about 48-49.5% and a particle size of about 3-15 µm.

Polyethylene oxide (PEO) and polyethylene glycol (PEG) as used herein are polyether compounds represented by the following general formula: 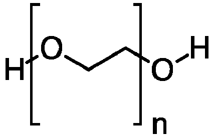 Wherein PEG is commonly referred to polymers having a molecular mass below 20.000 g/mol and PEO commonly refers to polymers having a molecular mass above 20.000 g/mol. Used together for preparing the coat layer(s), PEO and PEG may be mixed in different ratios, such as for example 10/90, 20/80, 30/70, 40/60 or 50/50 ratio and may have a molecular mass of about and between 4000 - 7000000 g/mol, preferably about 100.000 g/mol for PEO and about 4000 g/mol for PEG.

Basic butylated methacrylate (co)polymer is a cationic (co)polymer based on dimethylaminoethyl methacrylate, butyl methacrylate and methyl methacrylate, and is commonly known under the Tradename Eudragit® E PO. Polyvinylcaprolactam (PVCAP) as used herein represents polyvinyl-substituted caprolactam, wherein caprolactam is represented by the following formula:

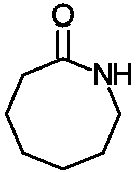

Polyvinylacetate as used herein is represented by the following general formula:

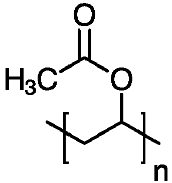

The copolymer polyvinylcaprolactam - PEG - polyvinylacetate as used in this invention is generally known under the Tradename Soluplus® and is represented by the following general formula:

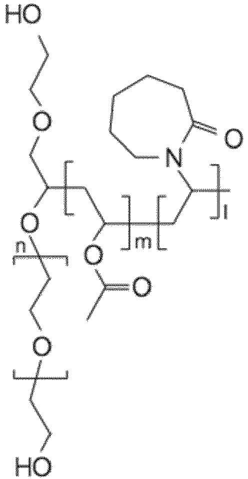

The co-extrusion process for preparing the multi-layered release formulation according to this invention, can generally be described as follows. A first composition comprising at least one of an ethylcellulose or a polycaprolactone; and a second composition comprising at least one (co)polymer as defined herein are provided; wherein at least one, and preferably at least two of said compositions further comprises an active ingredient. Said compositions are provided by mixing the aforementioned components supplied in the amounts as specified herein. Said components may be in the form of particles, but are preferably in powdered form and may optionally further be mixed with one or more additional components, such as but not limited to plasticizers and emulsifiers. Although in some embodiments of the invention the composition to be mixed into the co-extruder may contain liquid materials, dry feed is advantageously employed in the co-extrusion process of the present invention. The mixtures are fed in a co-extruder and passed through a heated area of the co-extruder at a temperature which will melt or soften the compositions. Typical co-extrusion melt temperatures are from about 60°C to about 160°C. Preferably, when polycaprolactone is used for preparing the core layer, the co-extrusion melt temperature is about 70°C - 80°C, more in particular 70°C; furthermore when ethylcellulose is used for preparing the core layer, preferably the co-extrusion melt temperature is about 120°C - 150°C, more preferably about 130°C - 140°C. The operating temperature range should be selected which will minimize the degradation or decomposition of the active ingredient and any other components of the composition during processing.

The molten or softened mixtures then exit via the die, or other such element, at which time the mixtures (now called the co-extrudate) begin to harden. The co-extrudate can exit the co-extruder in various shapes, such as a film, sheet, rods, strands or other cross sections. Since the co-extrudate is still warm or hot upon exiting the co-extruder, it can be easily shaped or molded into various shapes, for example into a film, chopped, ground to powders, spheronized into beads or pellets, cut into strands, tableted or otherwise processed to the desired physical form by methods well known to the skilled person. For example, the co-extrudate can be processed to various solid pharmaceutical dosage forms by comminuting the co-extrudate in the shape of a film, sheet or strands into various forms, such as pellets, beads, granules or powders with known means, such as pelletizing, grinding or milling, and converting the particles to a dosage form. If a multilayered film is to be produced, the molded film can be combined with other film layers while it is still warm or hot or after it has been cooled down.

In a preferred embodiment, the shape of the co-extrudate is a rod or cylinder. The core layer of said co-extrudate preferably has a diameter in the range of 0.1 to 5 mm, in particular of 0.1 to 3 mm, preferably 3 mm. The coat layer(s) of the co-extrudate according to this invention preferably taken together have a thickness of 0.1 to 3 mm, in particular 0.5 to 1 mm, preferably 0.5 mm. The height of the co-extrudate according to this invention is 1 - 6 mm, preferably 1-3 mm, more preferably 2 or 3 mm. The total dimensions of the co-extrudate are preferably 4x2 mm.

The dosage form is formulated for oral or buccal drug delivery, and in particular, is for oral delivery for release of the active agent into the gastrointestinal tract. Preferably, the release formulation of the present invention is shaped as or incorporated into solid dosage forms for oral administration such as but not limited to tablets, pills and capsules. In a specific embodiment, the multi-layered release formulation is a multi-particulate form, meaning that it consists of a multiplicity of small discrete units, each exhibiting some desired characteristics. In these systems, the dosage of the drug substance(s) or active ingredient(s) is divided in a plurality of subunits, typically consisting of particles with a diameter of 0.05-6.00mm; in particular with a diameter of 0,05 to 1, 2, 3, 4, 5 or 6mm. Thus multi-particulate dosage forms are pharmaceutical formulations in which the active substance is present as a number of small independent subunits. Pellets typically belong to the multi-particulate drug delivery forms. Multi-particulates are less dependent on gastric emptying rate, have a lower tendency for local irritation and have a reduced risk of dose dumping. Other processes such as injection molding, hot dipping, melt casting, solution casting and compression molding used for producing mono-layered or multilayered films or for producing particles are disclosed. By using any of these methods, the composition may be shaped as needed according to the desired mode of administration, for example films, tablets, pills, lozenges, and capsules.

If desired, the co-extruded composition of the present invention, can be combined with pharmaceutical excipients to produce pharmaceutical dosage forms, such as one or more fillers, pigments, colorants, flavorants, disintegrating agents, binders, plasticizers, antioxidants, lubricants, solid diluents and/or liquid diluents. Examples of useful liquid diluents are oils, water, alcohols, or mixtures thereof, with or without the addition of pharmaceutically suitable surfactants, suspending agents, or emulsifying agents.

The active ingredient that may be administered using the formulations, systems and methods of the invention are not limited, as the invention enables the effective delivery of a wide variety of active agents. The term "active agent/ingredient or drug" as used herein refers to therapeutic, diagnostic, cosmetic or prophylactic pharmaceutical and veterinary agents as well as other agents. The active ingredient is present in at least one of the core and/or coat layer(s), but preferably is present in both or all layers of the dosage form. In case both the core and coat layer(s) contain an active ingredient, said ingredient may be the same for each layer, but may also be different between the different layers.

The therapeutic or active agent may be selected from any of the various classes of such agents including, but not limited to, analgesic agents, anesthetic agents, anti-anginal agents, antiarthritic agents, anti-arrhythmic agents, antiasthmatic agents, antibacterial agents, anti-BPH agents, anticancer agents, anticholinergic agents, anticoagulants, anticonvulsants, antidepressants, antidiabetic agents, antidiarrheals, anti-epileptic agents, antifungal agents, antigout agents, antihelminthic agents, antihistamines, antihypertensive agents, antiinflammatory agents, antimalarial agents, antimigraine agents, antimuscarinic agents, antinauseants, antineoplastic agents, anti-obesity agents, antiosteoporosis agents, antiparkinsonism agents, antiprotozoal agents, antipruritics, antipsychotic agents, antipyretics, antispasmodics, antithyroid agents, antitubercular agents, antiulcer agents, anti-urinary incontinence agents, antiviral agents, anxiolytics, appetite suppressants, attention deficit disorder (ADD) and attention deficit hyperactivity disorder (ADHD) drugs, calcium channel blockers, cardiac inotropic agents, beta-blockers, central nervous system stimulants, cognition enhancers, corticosteroids, COX-2 inhibitors, decongestants, diuretics e.g. Hydrochlorothiazide (HCT), gastrointestinal agents, genetic materials, histamine receptor antagonists, hormonolytics, hypnotics, hypoglycemic agents, immunosuppressants, keratolytics, leukotriene inhibitors, lipid-regulating agents, macrolides, mitotic inhibitors, muscle relaxants, narcotic antagonists, neuroleptic agents, nicotine, nutritional oils, parasympatholytic agents, sedatives, sex hormones, sympathomimetic agents, tranquilizers, vasodilators, vitamins, and combinations thereof.

Active agents according to the invention also include nutrients, cosmeceuticals, diagnostic agents, and nutritional agents. Some agents, as will be appreciated by those of ordinary skill in the art, are encompassed by two or more of the aforementioned groups.

Anti-microbial agents such as broad spectrum antibiotics for combating clinical and sub-clinical infection, for example gentamycin, vancomycine and the like are also appropriate. Other suitable therapeutic agents are naturally occurring or synthetic organic or inorganic compounds well known in the art, including non-steroidal anti-inflammatory drugs, proteins and peptides (that may be produced either by isolation from natural sources or through recombination), hormones (for example androgenic, estrogenic and progestational hormones such as oestradiol), bone repair promoters, carbohydrates, antineoplastic agents, antiangiogenic agents, vasoactive agents, anticoagulants, immunomodulators, cytotoxic agents, antiviral agents, antibodies, neurotransmitters, oligonucleotides, lipids, plasmids, DNA and the like.

Suitable therapeutically active proteins include e.g. fibroblast growth factors, epidermal growth factors, platelet-derived growth factors, macrophage-derived growth factors such as granulocyte macrophage colony stimulating factors, ciliary neurotrophic factors, tissue plasminogen activator, B cell stimulating factors, cartilage induction factor, differentiating factors, growth hormone releasing factors, human growth hormone, hepatocyte growth factors, immunoglobulins, insulin-like growth factors, interleukins, cytokines, interferons, tumor necrosis factors, nerve growth factors, endothelial growth factors, osteogenic factor extract, T cell growth factors, tumor growth inhibitors, enzymes and the like, as well as fragments thereof.

Exemplary active agents that can be used in the formulations of the current invention include selective β1 receptor blockers used in the treatment of cardiovascular diseases, such a but not limited to metoprolol e.g. metroprolol tartrate (MPT), acebutolol, atenolol, betaxolol, and celiprolol; or non-steroidal anti-inflammatory drugs (NSAID) such as but not limited to aspirin, ketoprofen, ibuprofen, and natrium diclofenac.

As evident for a person skilled in the art, the load of the active ingredient(s) comprised in the pharmaceutical dosage form according to this invention, may vary depending on the active ingredient(s) used, the type of (co)polymer used, and the envisaged application area. In general, the core and coat layers may comprise between 1-60% wt (per layer) of active ingredient. In a specific embodiment the controlled release core layer may comprise 15-60% wt of active ingredient, more preferably 15%, 30%, 45%, 50% or 60%, and all values in between. In a specific embodiment the immediate release coat layer(s) may comprise between 1-30% wt of active ingredient, in particular 1.2%, 2.8%, 5.6%, 10%, 11.2%, 22.5%, 26% and 30%, and all values in between.

As already indicated before, the (co)polymer used for preparing the coat layer(s) of the multi-layered release formulation according to this invention, in combination with caprolactone and/or ethylcellulose as used for the core layer, fulfill one or more of following requirements:
- they are mutually compatible,
- they have similar rheological properties, so that they can be co-extruded under the same conditions of pressure and temperature,
- during the co-extrusion process, the coat layer(s) should remain separate from the core layer, but still sufficiently adhere thereto,
- the used (co)polymers should have the desired drug release profile.

Compatibility of polymers is defined as the capability of the individual component substances to exhibit interfacial adhesion. Compatibility is often established by the observation of mechanical integrity under the intended conditions of use of a composite or an immiscible polymer blend.

The polymers should be extrudable at similar extrusion temperatures and have similar viscosities (not too solid, but also not too liquid) at that temperature because they need to flow through the co-extrusion die under the same temperature conditions. The viscosity of a polymer in function of temperature and shear can be measured using a melt flow indexer.

The adhesion between the layers should be high enough to avoid separation of the layers during downstream processing. The adhesion is measured using a tensile tester: the sliced co-extrudates are placed on a holding device with a central opening and are positioned in such a way that only the coat is supported by the device, while the core is placed over the central opening. Using a probe, which applies a downward force on the core, the maximum force needed to separate the core from the coat is measured.

The polymers in the co-extrudates are chosen according to the desired release rate. The drug release rate is tested during drug dissolution tests as e.g. described in the present examples. The (co)polymers for preparing the coat layer(s) according to this invention, are selected from the group consisting of a combination of polyethylene oxide (PEO) and polyethylene glycol (PEG; Basic Butylated Methacrylate (co)polymer; a (co)polymer of polyvinylcaprolactam, PEG and polyvinylacetate; and combinations thereof.

In a further embodiment, the multi-layered release formulation according to this invention, further comprises one or more, i.e. 2, 3, 4 or 5, additional core and/or coat layers, wherein at least two, i.e. 2, 3, 4, 5, or more layers comprise an active ingredient.

In a preferred embodiment, the present invention provides a solid pharmaceutical oral dosage form according to this invention, wherein the core layer comprises at least one polycaprolactone; and the coat layer(s) comprises at least one (co)polymer selected from the group consisting of a combination of polyethylene oxide and polyethylene glycol; Basic Butylated Methacrylate (co)polymer; a (co)polymer of polyvinylcaprolactam, PEG and polyvinylacetate; and combinations thereof. More specific, the present invention provides a solid pharmaceutical dosage form wherein the core layer comprises polycaprolactone and the coat layer(s) comprises polyethylene oxide and polyethylene glycol.

In yet another preferred embodiment, the present invention provides a solid pharmaceutical oral dosage form according to this invention, wherein the core layer comprises at least one ethylcellulose and a plasticizer and/or PEO; and the coat layer(s) comprises at least one (co)polymer selected from the group consisting of a combination of polyethylene oxide (PEO) and polyethylene glycol (PEG); Basic Butylated Methacrylate (co)polymer; a (co)polymer of polyvinylcaprolactam, PEG and polyvinylacetate; and combinations thereof. More specific, the present invention provides a solid pharmaceutical dosage form wherein the core layer comprises ethylcellulose and a plasticizer and/or PEO, and the coat layer(s) comprises a (co)polymer of polyvinylcaprolactam, PEG and polyvinylacetate, or alternatively, wherein the coat layer(s) comprises a Basic Butylated Methacrylate (co)polymer, or as a further alternative wherein the coat layer(s) comprises polyethylene oxide and polyethylene glycol.

The core layer(s) of the solid pharmaceutical dosage form according to this invention may comprise between 20 - 99,9% weight of polycaprolactone and/or ethylcellulose.

The coat layer(s) of the solid pharmaceutical dosage form according to this invention may comprise between 10 - 99,9% weight of (co)polymers as defined herein. In particular, the coat layer(s) comprise between 30 - 99,9%, more in particular between 35 - 99,9% , and even more in particular between 40 - 99,9% weight of the (co)polymers as defined herein.

The coat and/or coat layer(s) may further comprise at least one of a plasticizer.

Plasticizers are generally used to increase the plasticity or fluidity of the material to which they are added. Suitable plasticizers are preferably biodegradable such as for example alkyl citrates including TEC (triethyl citrate). Alternatively, hydrophobic plasticizers such as dibutyl sebacate (DBS) and phthalates may also be used. The dosage form of the invention may comprise between 1 - 30% weight of a plasticizer, preferably 5%, 10%, 15%, 20%, 25% or 30% of a plasticizer.

The pharmaceutical dosage form according to this invention may be in any suitable administration form capable of comprising a multi-layered release formulation. For example, the multi-layered release formulation may be compressed during a tableting process providing a tablet or any other compressed dosage form. Alternatively the multi-layered release formulation may be encapsulated. Preferably the pharmaceutical dosage form according to this invention is an oral dosage form.

In a further aspect, the present invention provides a solid pharmaceutical dosage form as defined herein for use as a medicament, in particular for the bi-phasic or multi-phasic release of one or more active ingredients.

Furthermore, the present invention provides a method for the bi-phasic or multi-phasic release of one or more active ingredients; said method comprising administering to a patient in need thereof a solid pharmaceutical dosage form as defined herein.

The present invention also provides a method for preparing a multi-layered release formulation according to this invention; said method comprising co-extruding:
- at least one core layer comprising at least one polymer selected from polycaprolactone, ethylcellulose, or combinations thereof, and when the polymer is ethylcellulose the core layer contains at least one plasticizer; and
- at least one coat layer comprising at least one (co)polymer selected from the group consisting of: a combination of polyethylene oxide and polyethylene glycol; Basic Butylated Methacrylate (co)polymer; a (co)polymer of polyvinylcaprolactam, PEG and polyvinylacetate; and combinations thereof.

In a preferred embodiment of said method, at least two layers, preferably the core and the coat layer, comprise an active ingredient.

Finally, the present invention provides a multi-layered release formulation obtainable by a process as defined herein.

This invention will be better understood by reference to the Experimental Details that follow, but those skilled in the art will readily appreciate that these are only illustrative of the invention as described more fully in the claims that follow thereafter. Particular embodiments and examples are not in any way intended to limit the scope of the invention as claimed.

### EXAMPLES

### REFERENCE EXAMPLE 1: Selection of suitable polymers for immediate release

In this first example, we examined the release profiles of multiple (co)polymers, to determine the most suitable (co)polymers for preparing a solid pharmaceutical dosage form having an immediate release coat layer.

### 1. Copolymer comprising polyvinylcaprolactam, PEG and polyvinylacetate (SOLUPLUS^{®})

To evaluate the release properties, formulations with different drug loads (2.5, 5, 10% HCT (hydrochlorothiazide) (w/w)) were tested. The influence of the addition of a plasticizer, Pluronic® F68 (copolymer of ethylene and propylene oxide), was also tested by making formulations with a fixed drug load of 5% HCT and various amounts of this plasticizer (0, 10, 20% Pluronic® F68). All formulations were extruded at a temperature of 130 °C, except the one without Pluronic® F68 that was extruded at 150 °C. The addition of Pluronic® F68 significantly lowered the extruder torque. The extrudates that contained 2.5% drug were transparent, indicating the formation of a solid solution, where the drug is present in the amorphous state. The extrudates with 10% drug were opaque meaning that the drug remained in the crystalline state.

Subsequently the dissolution profile of the particles was determined by exposing them to a 0.1M solution of hydrochloric acid (pH=1, mimicking the pH of the stomach) in the dissolution bath. The equipment consisted of a VK 7010 dissolution system combined with a VK 8000 automatic sampling station (VanKel Industries, NJ, USA). All vessels were filled with 900 ml of the dissolution solution. Sink conditions were maintained during the experiments. The bath temperature was kept constant at 37 ±0.5 °C. The rotational speed of the baskets was set to 100 rpm. Samples (5 ml) were withdrawn at 5, 10, 15, 20, 30, 45 and 60 minutes and were analyzed spectrophotometrically at 272 nm using a Perkin-Elmer Lambda 12 UV-VIS double beam spectrophotometer (Zaventem, Belgium).

After one hour in the dissolution bath, the particles that contained only 2.5% drug were almost completely dissolved. Those with more drug didn't dissolve at all. They tended to swell and became white. These observations were in agreement with the release results. The particles that almost dissolved in one hour released 80% of drug in one hour (see figure 1). Those with higher drug loads didn't dissolve, resulting in an incomplete drug release (<30% in 1 h). Furthermore, with an increasing drug load, the relative release (%) decreased. It was also seen that the level of Pluronic® F68 didn't affect the drug release.

### 2. Copolymer of EUDRAGIT® E PO

Three formulations with different drug loads (10, 20, 30% HCT) were tested. No plasticizer was added to any of the formulations. They were all extruded at a temperature of 140 °C which resulted in torque values of 60-70%. After cutting into length the particles were placed in the dissolution vessels. The release results were very promising. The particles disintegrated fast, due to the fact that this polymer dissolves in acidic media. The drug release rate from all three formulations was very high reaching 100% of release in only 30 minutes (see figure 2a). The drug load had no considerable effect on the release rate.

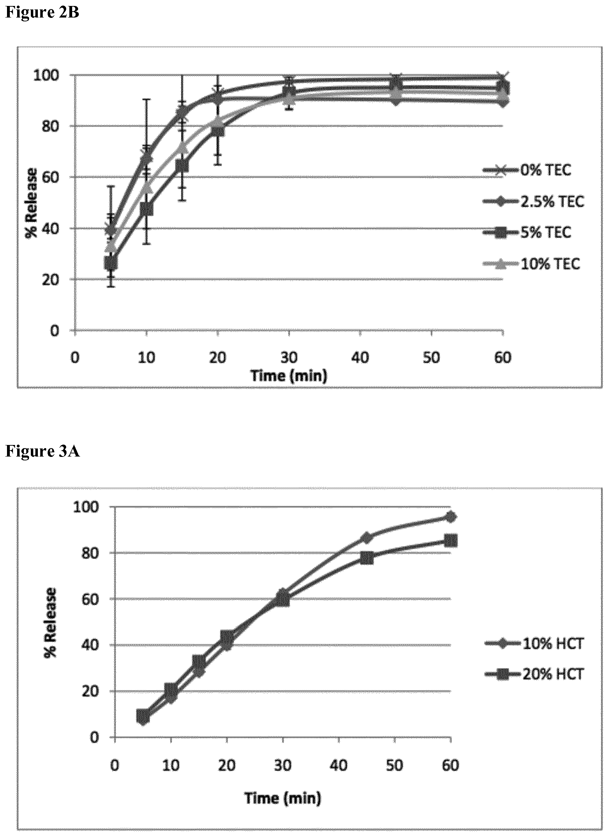

In a second phase, the influence of the plasticizer triethylcitrate (TEC) on drug release was investigated. Different amounts of triethylcitrate were added to a formulation with a fixed drug load of 10%. It was found that there was no considerable effect, irrespective the amount that was added (see figure 2b). As a result, this polymer was found to be very useful as the coat for co-extrusion.

### 2. Polymer of polyethylene oxide and polyethylene glycol

Two formulations with different drug loads (10 and 20% HCT) were extruded at the same temperature (90 °C) resulting in torque values of 50%. The diameter of the resulting extrudates was larger than that of the die, indicating that there was extrudate swell. The drug release from both formulations was very similar. The release was completed in 1 hour, but the release rate was rather low (see figure 3a).

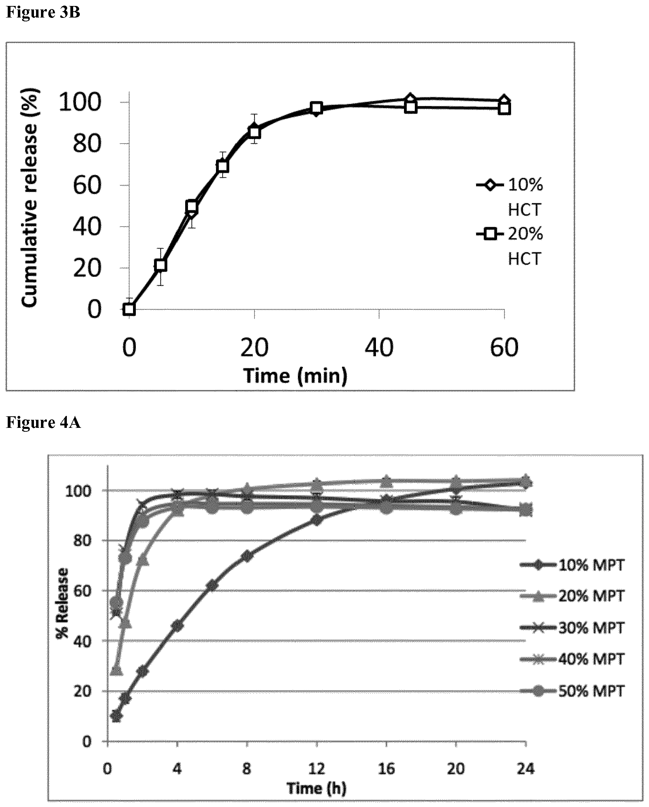

In order to increase the release rate, polyethylene glycol with a molecular weight of 4000 was added to the formulation. This resulted in a faster drug release that could be used for further studies (see figure 3b).

### REFERENCE EXAMPLE 2: Selection of suitable polymers for controlled release

In this second example, we examined the release profiles of multiple (co)polymers, to determine the most suitable (co)polymers for preparing a solid pharmaceutical dosage form having a controlled release core layer.

### 1. Copolymer comprising Eudragit® RS PO

Five formulations with different drug loads up to 50% were extruded. In a first phase, no plasticizer was added to the formulations. The extrusion temperature was 120 °C for the formulations with 10 and 20% of drug (MPT - metoprolol tartrate), 110 °C for the one with 30% drug and 100 °C for the ones with 40 and 50% of drug. The torque was as follows: 75% for 10% MPT, 65% for 20% MPT, 60% for 30% MPT, 70% for 40% MPT and 60% for 50% MPT. The extrudates were glassy and transparent up to 30% of drug. Extrudates with higher drug loads became slightly white and were more flexible.

After 24 hours of dissolution, all particles retained their shape but became white. The drug release from the extrudate with only 10% MPT was relatively slow compared to the one with 20% of drug (see figure 4a). Formulations with 20% MPT or more showed a burst release, reaching 100% of release in less than 4 hours. No sustained release formulations were obtained.

Multiple attempts to optimize the formulations in order to obtain a suitable sustained release formulation all failed (data not shown). These attempts included:
- the addition of the plasticizer triethylcitrate (TEC) → the addition of TEC had no significant effect on the release of MPT from these matrix particles.
- the addition of the hydrophobic substance dibutylsebacate (DBS), in order to retard the release → the addition of at least 9% (w/w) DBS to the polymer resulted not only in a slower but also an incomplete drug release, furthermore, extrudates with 9% DBS or more turned out to be unstable during storage.
- the addition of the hydrophobic substance Ethocel® std 10 (ethylcellulose) → different amounts up to 50% were tested, but the release remained too fast.
- the addition of clays → the addition of Kaolien turned out to have no delaying effect at all.
- the variation of particle size → the bigger the particles, the slower the drug was released. However, even for the biggest particles almost 60% of drug was already released in 4 hours.
- increase of the diameter of the particles → 3 different die diameters were tested (2, 3, 4 mm). With the 2 mm die, there was no extrudate swell at all. The swelling appeared with the 3 mm die and was worst with the 4 mm die.

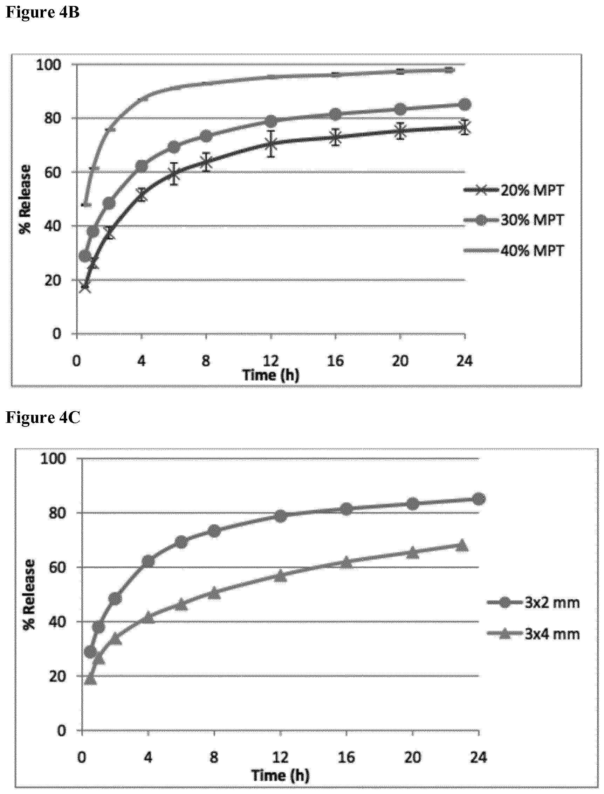

These preliminary experiments showed that two factors had a major influence on the release of MPT from these Eudragit® RS PO matrices. These two factors were the drug load, the particle size and the amount dibutylsebacate added to the formulation. Some extra experiments were done to confirm these findings and to gain more information. Formulations with a fixed amount of DBS (10% (w/w) to the polymer), but different drug loads (20, 30, 40% MPT) were extruded at a temperature of 100 °C. The dissolution results revealed that there were significant differences in the total amount of MPT released in 24 h (see figure 4b).

Furthermore, two different particle sizes of the same formulation with 30% MPT and 10% DBS were compared (3x4 mm versus 3x2 mm). For this small difference in size, there was a huge difference in release profile. As expected, the small particles had a higher release rate in the first 4 h than the bigger ones (see figure 4c). Moreover the release of MPT from these small particles was more or less completed in 24 h, whereas for the bigger ones it was not the case.

### 2. Copolymer comprising Eudragit® NE 30D

The molecular structure of Eudragit® NE is similar to that of Eudragit® RS except for the fact that the structure of Eudragit® NE doesn't contain quaternary ammonium groups. It was thought that, due to the lack of these ionic groups, the water uptake and swelling of this polymer would be less than for Eudragit® RS, resulting in a slower release of MPT.

Since Eudragit® NE was not available as a powder, the dispersion (Eudragit® NE 30D) was freeze dried. The dispersion contained not only the polymer but also 1,5% of nonoxynol as a surfactant. After freeze drying for 4 days, the obtained product was manually grinded into a fine powder and mixed with the drug, creating a formulation with 30% MPT. This mixture was extruded at two temperatures, 100 °C and 90 °C. The torque was 50% and 70% respectively. When extrusion was performed at 100 °C, extrudate swelling was observed while extrusion at 90 °C resulted in shark skinning of the extrudate.

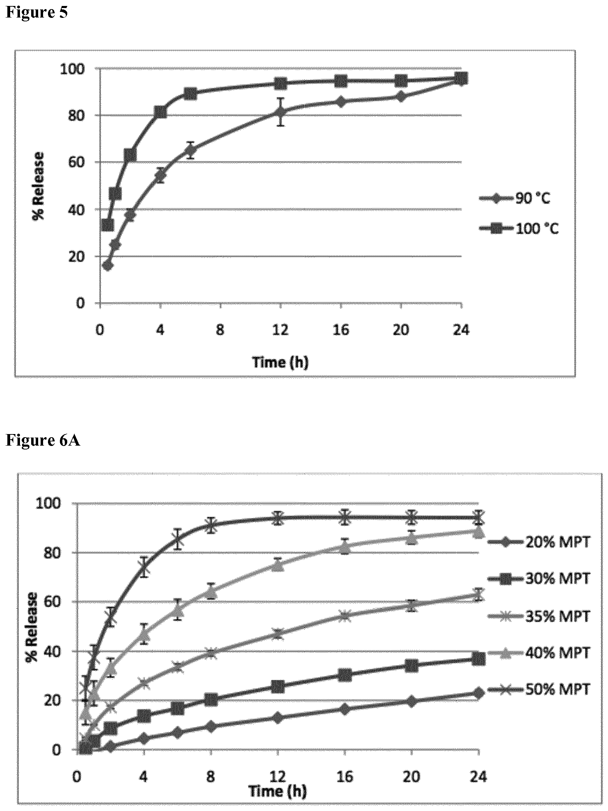

Comparison of the release profiles pointed out that the release rate during the first 4 h was much higher for the extrudates produced at 100 °C than for those extruded at 90 °C (see figure 5). Probably this difference was caused by the fact that the extrudates extruded at 100 °C were swollen and thus more porous, resulting in a faster drug release. Anyhow these release profiles, especially the one of the extrudate produced at 90°C, were better than those obtained with Eudragit® RS PO, but were still not good enough to provide a sufficient sustained drug release. Due to the fact that there were also other drawbacks (freeze drying step, shark skinning of the extrudate), this polymer was not used for co-extrusion.

### 3. Polymer comprising Capa 6506 (polycaprolacton)

As a preliminary experiment formulations with different drug loads (20, 30, 35, 40, 50% (w/w) MPT) were extruded. The extrusion temperature was 80 °C for each formulation, resulting in torque values of 50-60%. The resulting extrudates were all flexible and white.

The results of the dissolution test showed that for formulations with less than 40% MPT the drug release was incomplete (see figure 6a). The release of MPT from the formulation that contained 50% was too fast. The total amount of drug was already released in 8 h. The only formulation that gave an acceptable sustained release profile was the one with 40% MPT.

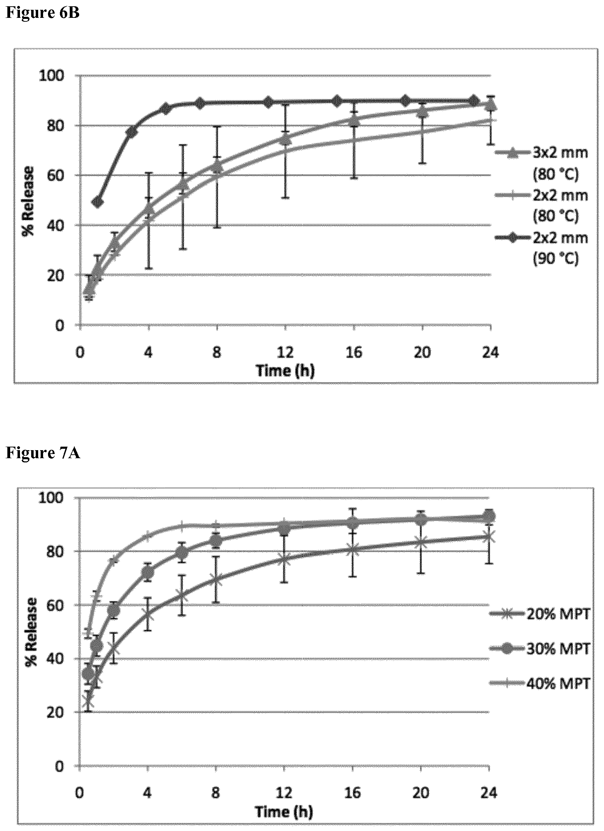

To have an idea of the influence of the extrusion temperature and particle size on drug release two extra formulations were extruded with a size of 2x2 mm. One formulation was extruded at a temperature of 90 °C, unlike the reference that was extruded at 80 °C. This small test showed immediately that the influence of the reduction in particle size was very small (see figure 6b). The extrusion temperature on the other hand had a major influence. An increase of only 10 °C resulted in an unacceptable fast drug release.

### 4. Polymer comprising Ethocel® STD 10 (ethylcellulose)

Before mixing with the drug, this polymer was plasticized with 20% of DBS. As a screening, formulations with three different drug loads (20, 30, 40% (w/w) MPT) and two different particle sizes (3x2 mm and 2x2 mm) were compared. All formulations were extruded at a temperature of 110°C resulting in torque values of 60-70%.

The release of the small (2x2 mm) particles was too fast (see figure 7a). The higher the drug load, the higher the release rate was. The formulation with 40% MPT reached 100% release in only 6 h. The one with 20% MPT was the best in terms of release, but a drug load of 20% is very low.

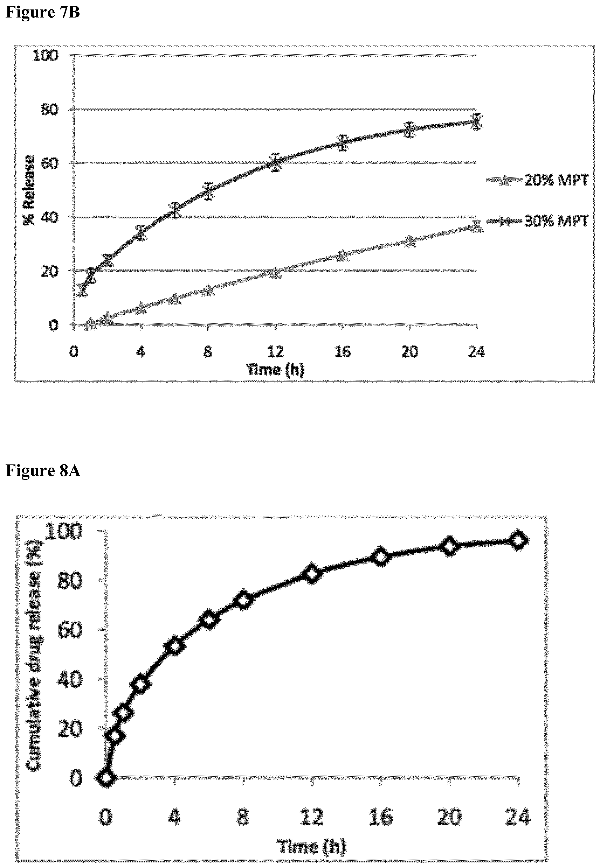

Compared to the smaller particles, the particles with a size of 3x2 mm released the drug slower. The formulation with 20% MPT showed incomplete drug release (<40% in 24h), the one with 30% MPT showed an acceptable sustained release profile (see figure 7b). This last formulation is very suitable for co-extrusion.

### EXAMPLE 3: Preparation and testing of co-extruded two-layered pharmaceutical dosage forms

In this example, we manufactured different co-extruded two-layered pharmaceutical dosage forms comprising an immediate release coat layer and a controlled release core layer, based on the findings as represented in examples 1 and 2.

As already explained herein before, the pharmaceutical dosage form according to this invention preferably provides a first burst off phase in which the majority of the active ingredient(s) comprised in the immediate release coat layer(s) of the formulation is released within the first hour after administration of the pharmaceutical dosage form, preferably within the first 30 min, most preferably within the first 20 min. In the second release phase, the majority i.e. about 80, 85, 90, 95 or 100% of the active ingredient(s) comprised in the controlled release core layer, is gradually released within 24 hours after administration of the pharmaceutical dosage form.

In particular, the solid pharmaceutical dosage form as described herein provides a first burst off phase in which about 10-50%; preferably about 20-40%; most preferably about 30% of the total amount of active ingredient in the formulation is released within the first hour after administration of the pharmaceutical dosage form, preferably within the first 30 min, most preferably within the first 20 min. In particular, nearly all of the remainder of the drug, i.e. more than 80%, is gradually released within 24 hours after administration of the pharmaceutical dosage form.

Taken together, the pharmaceutical dosage forms according to this invention preferably provide a release profile wherein up to about 30% of the total amount of active ingredient(s) is released within the first 20 min, up to about 80% of the total amount of active ingredient(s) is released within the first 16 - 20 hours and up to about 90-100% of the total amount is released within 24 hours after administration of the pharmaceutical dosage form.

### 1. Formulation 1 - Polycaprolacton core

Composition of co-extrudates:
- Core layer: controlled release formulation of 45% MPT (metoprolol tartrate) in polycaprolacton (Capa)
- Coat layer: immediate release formulation of 10% HCT (hydrochlorothiazide) in PEO 100 000 / PEG 4000 (1/1)
Dimensions of co-extrudates:
- diameter of core: 3 mm
- thickness of coat: 0.5 mm
- heigth of particles: 2 mm
- total dimensions of particles: 4 x 2mm
Extrusion parameters:
- Temperature: 70°C
- Rotating speed: 60 rpm
- Feed speed: 250g/h (core), 150g/h (coat)

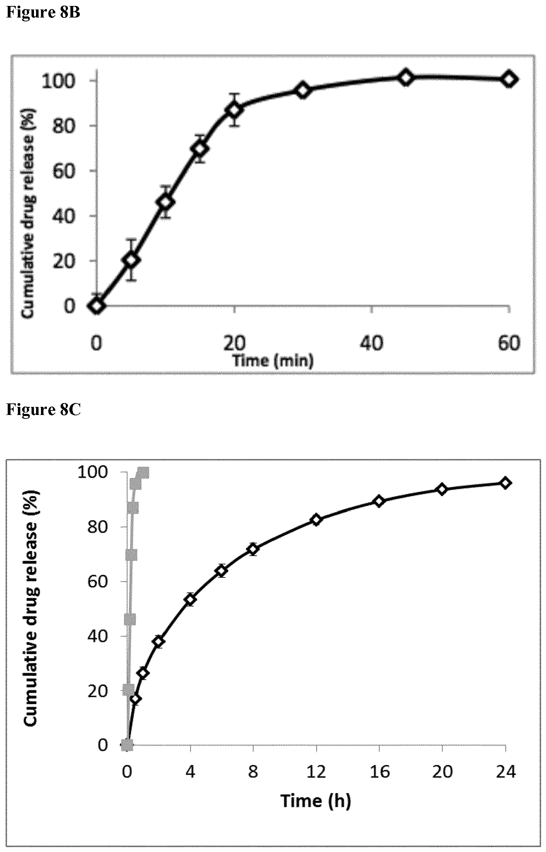

Results of the in vitro release profile are shown in figures 8a - 8c. As evident from figure 8c, the desired profile wherein the majority (about 100%) of the active ingredient (HCT) contained in the coat layer is released very fast (within the first 20 min), and the majority (about 95%) of the active ingredient (MPT) contained in the core layer is released within 24h, was obtained.

### 2. Formulation 2 - Polycaprolacton core

Composition of co-extrudates:
- Core layer: controlled release formulation of 50% NaD (natrium diclofenac) in polycaprolacton (Capa)
- Coat layer: immediate release formulation of 22.5% NaD (natrium diclofenac) in PEO 100 000 / PEG 4000 (1/1)
Dimensions of co-extrudates:
- diameter of core: 3 mm
- thickness of coat: 0.5 mm
- heigth of particles: 2 mm
- total dimensions of particles: 4 x 2mm
Extrusion parameters:
- Temperature: 70°C (die on 80°C)
- Rotating speed: 60 rpm
- Feed speed: 150g/h (coat + core)

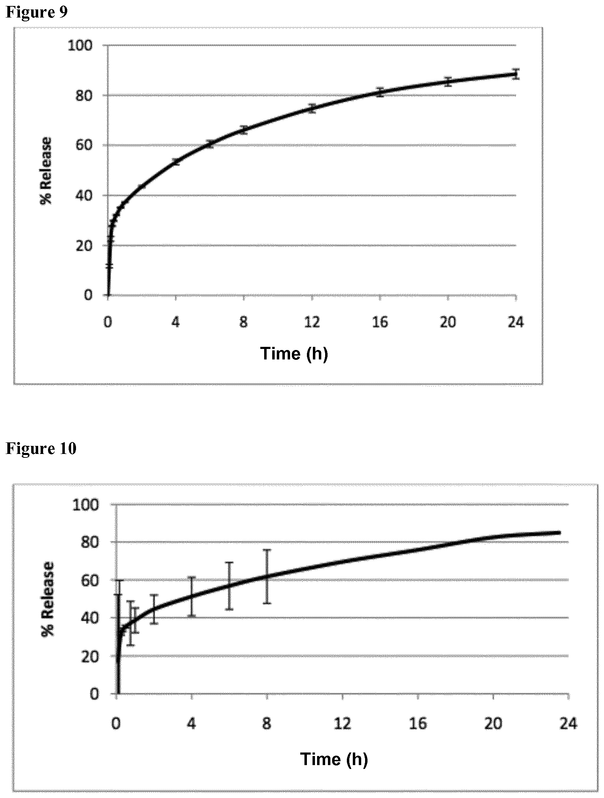

Results of the in vitro release profile are shown in figure 9. As evident from figure 9, the desired profile wherein about 30% of the total load of active ingredient (NaD) is released very fast (within the first 20 min), and most of the remainder of the drug (about 90% of the total load) is released within 24h, was obtained.

### 3. Formulation 3 - Ethylcellulose core

Composition of co-extrudates:
- Core layer: controlled release formulation of 60% NaD (natrium diclofenac) in ethylcellulose + 20% dibutyl sebacate (DBS)
- Coat layer: immediate release formulation of 26% NaD (natrium diclofenac) in Soluplus® + 10% Pluronic® F68
Dimensions of co-extrudates:
- diameter of core: 3 mm
- thickness of coat: 0.5 mm heigth of particles: 2 mm
- total dimensions of particles: 4 x 2mm
Extrusion parameters:
- Temperature: 140°C
- Rotating speed: 60 rpm
- Feed speed: 200g/h (coat + core)

Results of the in vitro release profile are shown in figure 10. As evident from figure 10, the desired profile wherein about 30% of the total load of active ingredient (NaD) is released very fast (within the first 20 min), and most of the remainder of the drug (about 85% of the total load) is released within 24h, was obtained.

### 4. Formulation 4 - Ethylcellulose core

Composition of co-extrudates:
- Core layer: controlled release formulation of 20% or 30% MPT (metoprolol tartrate) in ethylcellulose + 15% dibutyl sebacate (DBS)
- Coat layer: immediate release formulation of about 6% HCT (hydrochlorothiazide) in Basic Butylated Methacrylate (Eudragit ® E PO)
Dimensions of co-extrudates:
- diameter of core: 3 mm
- thickness of coat: 0.5 mm
- heigth of particles: 2 mm
- total dimensions of particles: 4 x 2mm
Extrusion parameters:
- Temperature: 130°C
- Rotating speed: 60 rpm
- Feed speed: 250g/h (core), 150g/h (coat)

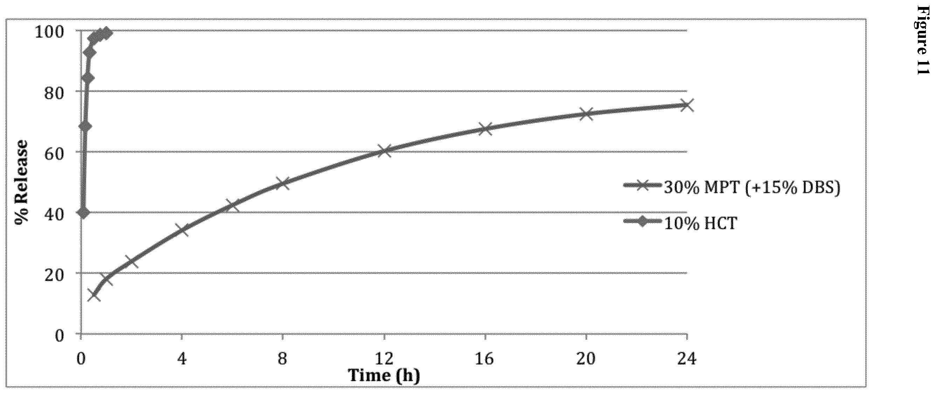

Results of the in vitro release profile are shown in figure 11. As evident from figure 11, the desired profile wherein the majority (about 100%) of the active ingredient (HCT) contained in the coat layer is released very fast (within the first 20 min), and the majority (about 80%) of the active ingredient (MPT) contained in the core layer is released within 24h, was obtained.

### 5. Formulation 5 - Ethylcellulose core

Composition of co-extrudates:
1.Formulation 5a:
   - Core layer: controlled release formulation of 30% MPT (metoprolol tartrate) in 62% ethylcellulose (EC) + 33% dibutyl sebacate (DBS) + 5% PEO 1M (Polyethylene oxide 1.000.000);
   - Coat layer: sustained release formulation of 5,6% HCT (hydrochlorothiazide) in 85% PEO 100.000 (Polyethylene oxide 100.000) + 15% PEG4000 (Polyethylene Glycol 4000)
2.Formulation 5b:
   - Core layer: controlled release formulation of 15% MPT in 53% EC + 27% DBS + 20% PEO 1M;
   - Coat layer: sustained release formulation of 2,8% HCT in 85% PEO 100.000 + 15% PEG4000
3.Formulation 5c:
   - Core layer: controlled release formulation of 30% MPT in 62% EC + 33% DBS + 5% PEO 1M;
   - Coat layer: sustained release formulation of 11,2% HCT in 85% PEO 100.000 + 15% PEG4000
Dimensions of co-extrudates:
- diameter of core: 3 mm
- thickness of coat: 0.5 mm
- heigth of particles: 2 mm
- total dimensions of particles: 4 x 2mm
Extrusion parameters:
- Temperature: 100°C (core + coat)
- Rotating speed: 150 rpm (core), 40 rpm (coat)
- Feed speed: 300 g/h (core), 200 g/h (coat)

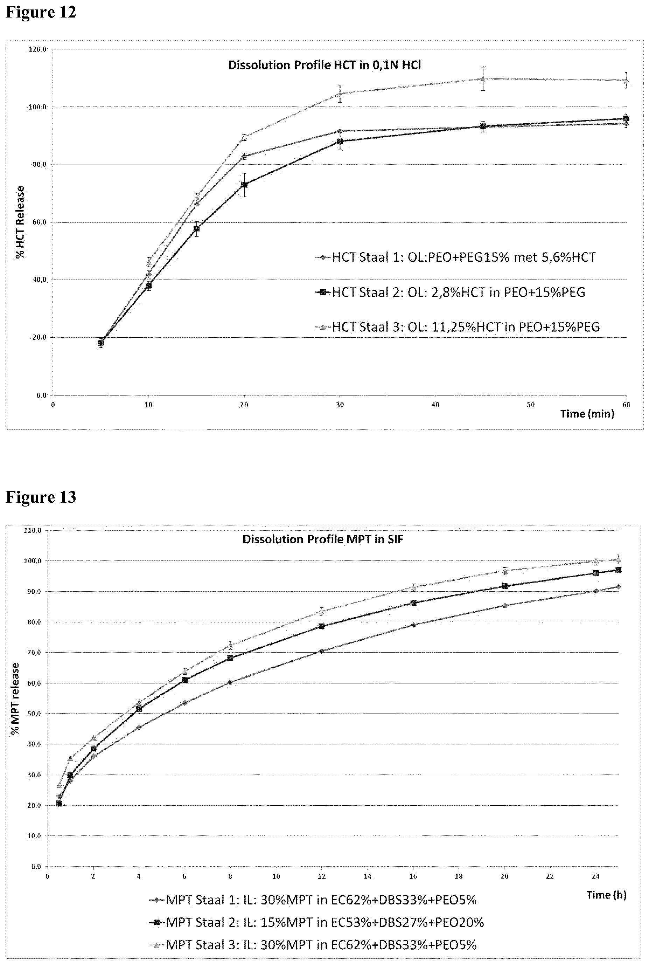

Results of the in vitro release profiles are shown in figures 12 and 13. As evident from figure 12, the desired profile wherein the majority (about 80-100%) of the active ingredient (HCT) contained in the coat layer is released very fast (within the first 20-30 min), and as evident from figure 13, the majority (about 80-100%) of the active ingredient (MPT) contained in the core layer is released within 24h, was obtained.

### EXAMPLE 4: In vivo study - Polycaprolactone core

### 4.1. Subjects and study design

A group of six male dogs (weight 23.5-39.0 kg) was used in this study. An oral dose of 200 mg metoprolol tartrate and 25 mg hydrochlorothiazide was administered to the dogs, either as experimental co-extruded mini-matrices or as reference formulation (Zok-Zid®, Pfizer, Brussels, Belgium). The core of the co-extrudate was formulated with 45% MPT, 54% polycaprolactone and 1% colloidal silicium dioxide, while the coat contained 10% HCT, 45% PEO and 45% PEG.

The mini-matrices of the experimental formulation were filled in hard-gelatin capsules, whereas the reference formulation was given as a tablet. The formulations were administered in randomized order with a wash-out period of at least 1 week between sessions. On the experimental day the dogs were fasted for 12 h prior to the study period, although water was available. Before administration of a formulation, a blank blood sample was taken. The formulations were orally administered with 20 ml water. The blood samples were collected in dry heparinized tubes at fixed time points: 0.25, 0.5, 0.75, 1, 1.5, 2, 3, 4, 5, 6, 8, 10 and 12 h after intake of the formulations. No food was administered to the dogs during the entire test period. Within 1 h after collection, blood was centrifuged for 10 min at 1500g. The plasma was separated and kept frozen at -20°C until analysis.

### 4.2. Metoprolol tartrate and hydrochlorothiazide assay

Metoprolol tartrate and hydrochlorothiazide plasma concentrations were determined using two different HPLC methods. For MPT, a validated HPLC fluorescence method was used [J. Fang, H.A. Semple, J. Song, Journal of Chromatography B,, 809 (2004) 9-14.]. Bisoprolol was used as internal standard. A solid phase extraction (SPE) procedure was used to extract metoprolol tartrate. Hydrochlorothiazide was determined using a validated HPLC-UV method [C. Vervaet, J.P. Remon, Pharm. Res.,14 (1997) 1644-1646.]. The drug was extracted from the plasma samples by means of liquid-liquid extraction with hydroflumethiazide as internal standard. Since no interfering peaks of HCT and MPT, respectively, the specificity of the methods was secured. An automatic integration system (software D-7000 Multi-Manager) was used for integration of the chromatographic peaks.

### 4.3. Data analysis

The peak plasma concentration (Cₘₐₓ), the extent of absorption (AUC₀₋₁₂ₕ) and the time to reach Cₘₐₓ (Tₘₐₓ) were calculated. The relative bioavailability (Frel, expressed in %) was calculated as the ratio of AUC₀₋₁₂ₕ between a test formulation and the reference formulation. Data were statistically analyzed using SPSS 17 (SPSS, Chicago, USA). To compare the effects of the different treatments, a paired samples t-test was performed with a significance level of α = 0.05.

### 4.4. Results

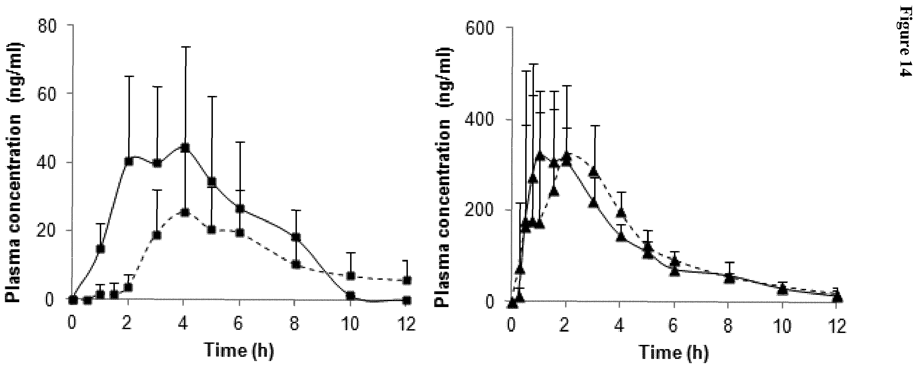

Figure 14 shows the mean plasma concentration-time profiles after oral administration of 200 mg MPT and 25 mg HCT as experimental mini-matrices and Zok-Zid® (2 tablets). Although Zok-Zid® is administered as a tablet, in contact with fluids, it immediately disintegrated into pellets, creating a multiparticulate system. According to the dissolution data, both test and reference formulation provided immediate release (less than 30 min) of HCT and sustained MPT release over 24h. The pharmacokinetic parameters of MPT and HCT are reported in table 1.

**Table 1: Mean pharmacokinetic parameters (±S.D.) of MPT and HCT after oral administration of 200 mg metoprolol tartrate and 25 mg hydrochlorothiazide to dogs (n=6), as experimental co-extruded mini-matrices (with a core consisting of 45% (w/w) MPT and a coat consisting of 10% (w/w) HCT) or as reference formulation (Zok-Zid®).**

| **MPT** | | | | **HCT** | | |
|---|---|---|---|---|---|---|
| | **Cₘₐₓ (ng/ml)** | **Tₘₐₓ (h)** | **AUC (ng.h/ml)** | **Cₘₐₓ (ng/ml)** | **Tₘₐₓ (h)** | **AUC (ng.h/ml)** |
| **Exp** | 73.6 ± 46.9 | 2.8 ± 0.7 | 345.2 ± 257.9 | 371.6 ± 126.0 | 1.6 ± 0.5 | 1353.8 ± 320.3 |
| **Ref** | 23.5 ± 19.7 | 4.2 ± 1.2 | 117.1 ± 95.6 | 459.9 ± 227.0 | 2.5 ± 1.1 | 1479.3 ± 346.5 |

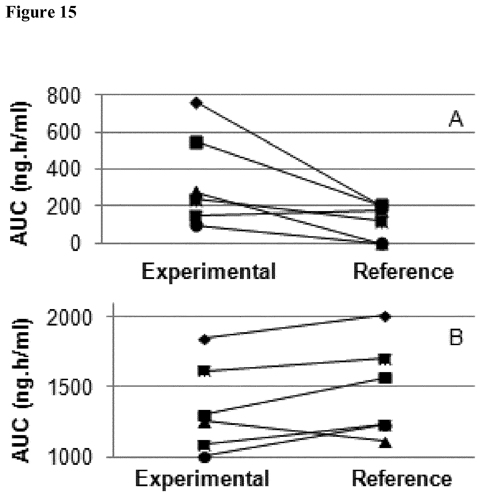

Figure 15 represents the AUC values of each dog separately after administration of the experimental and the reference formulation. The bioavailability data (Cₘₐₓ, Tₘₐₓ and AUC) of both formulations were comparable, without a statistical significant difference between the test and reference formulations (p > 0.05). Although there was a trend that the MPT bioavailability of the test formulation for each dog was higher than the reference (Fig. 15A), the difference was statistically not significant. While the coat of the co-extrudates dissolved during gastro-intestinal passage, intact cores (which still contained 6.6 ± 0.4% of the initial MPT dose) were recovered from the faeces of the dogs. As no swelling or erosion was observed while the pore size increased, release from the caprolactone core was diffusion-controlled.

### EXAMPLE 5: In vivo study - Ethylcellulose core

The in vivo experiment as defined in example 4, was repeated, thereby making use of the formulations 5a - 5c as defined in example 3 comprising 200 mg MPT and 25 mg HCT.

### Results

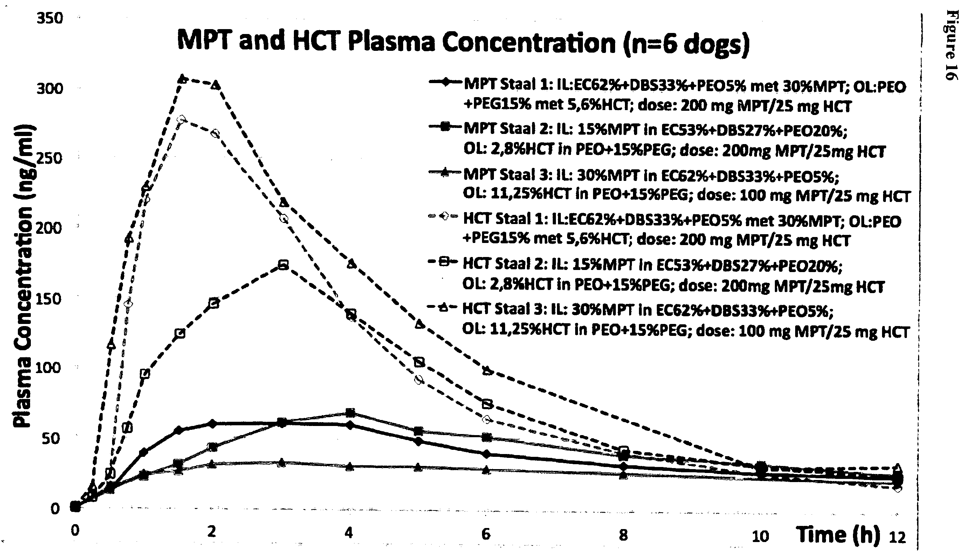

Figure 16 shows the mean plasma concentration-time profiles after oral administration of 200 mg MPT and 25 mg HCT as experimental mini-matrices According to the dissolution data, all formulations provided immediate release (less than 30 min) of HCT and sustained MPT release over at least 12h.

## Claims

1. A solid pharmaceutical oral dosage form, comprising a co-extrusion formed multi-layered release formulation, having:
- a core layer comprising at least one polymer selected from polycaprolactone, ethylcellulose, or combinations thereof, and when the polymer is ethylcellulose the core layer contains at least one plasticizer; and
- a coat layer comprising:
• a combination of polyethylene oxide and polyethylene glycol;
• Basic Butylated Methacrylate (co)polymer;
• a (co)polymer of polyvinylcaprolactam, PEG and polyvinylacetate; or
• combinations thereof; and
wherein at least one of said layers comprises an active ingredient.

2. The solid pharmaceutical oral dosage form according to claim 1, wherein the multi-layered release formulation further comprises one or more additional core and/or coat layers.

3. The solid pharmaceutical oral dosage form according to claims 1 or 2, wherein the core layer comprises at least one polycaprolactone.

4. The solid pharmaceutical oral dosage form according to any one of claims 1 to 3, wherein the core layer comprises at least one ethylcellulose.

5. The solid pharmaceutical oral dosage form according to any one of claims 1 to 4, wherein polycaprolactone is present at an amount of between 20 and 99,9 weight-% of the core layer.

6. The solid pharmaceutical oral dosage form according to any one of claims 1 to 3 and 5, wherein ethylcellulose is present at an amount of between 20 and 99,9 weight-% of the core layer.

7. The solid pharmaceutical oral dosage form according to any one of claims 1- 6, wherein the (co)polymer of the coat layer is present at an amount of between 10 and 99,9 weight-% of the coat layer.

8. The solid pharmaceutical oral dosage form according to any one of claims 1 to 7, wherein the core layer has a diameter in the range of 0.1 to 5 mm and wherein the coat layer(s) have a total thickness in the range of 0.1 to 3 mm.

9. The solid pharmaceutical oral dosage form according to any one of claims 1 to 8, wherein the core layer, and/or coat layer(s) further comprise at least one plasticizer.

10. The solid pharmaceutical oral dosage form of any one of claims 1- 9, wherein at least two of the layers of the multi-layered release formulation comprises an active ingredient.

11. The solid pharmaceutical oral dosage form according to any one of claims 1- 10, wherein each of the layers comprises an active ingredient.

12. The solid pharmaceutical oral dosage form according to any one of claims 1-11, wherein the core and the coat layer(s) comprise the same or a different active ingredient.

13. The solid pharmaceutical oral dosage form of any one of claims 1- 12, wherein the dosage form has a cylindrical shape.

14. The solid pharmaceutical oral dosage form of any one of claims 1- 13 for use as a medicament.

15. The solid pharmaceutical oral dosage form according to any one of claims 1-13, for use in the treatment of a disease by delivering one or more active ingredients to a subject in a biphasic or multi-phasic manner.

16. A method of preparing a multi-layered release formulation; said method comprising coextruding
- a core layer comprising at least one polymer selected from polycaprolactone, ethylcellulose, or combinations thereof, and when the polymer is ethylcellulose the core layer contains at least one plasticizer; and
- a coat layer comprising at least one (co)polymer selected from the group consisting of a combination of polyethylene oxide and polyethylene glycol; Basic Butylated Methacrylate (co)polymer; a (co)polymer of polyvinylcaprolactam, PEG and polyvinylacetate; and combinations thereof.

17. The method according to claim 16, wherein the co-extrudate is further shaped or molded into a shape selected from the group consisting of: a film, a sheet, pellets, beads, tablets, granules and powder.

## Patentansprüche

1. Feste pharmazeutische orale Dosierungsform, umfassend eine durch Co-Extrusion gebildete mehrschichtige Freisetzungsformulierung, welche aufweist:
- eine Kernschicht, umfassend mindestens ein Polymer, ausgewählt aus Polycaprolacton, Ethylcellulose oder Kombinationen davon, und, wenn das Polymer Ethylcellulose ist, enthält die Kernschicht mindestens einen Weichmacher; und
- eine Überzugsschicht, umfassend:
• eine Kombination von Polyethylenoxid und Polyethylenglykol;
• basisches butyliertes Methacrylat-(Co-)Polymer;
• ein (Co-) Polymer von Polyvinylcaprolactam, PEG und Polyvinylacetat; oder
• Kombinationen davon; und
wobei mindestens eine der Schichten einen aktiven Bestandteil umfasst.

2. Feste pharmazeutische orale Dosierungsform nach Anspruch 1, wobei die mehrschichtige Freisetzungsformulierung ferner eine oder mehrere zusätzliche Kern- und/oder Überzugsschichten umfasst.

3. Feste pharmazeutische orale Dosierungsform nach Anspruch 1 oder 2, wobei die Kernschicht mindestens ein Polycaprolacton umfasst.

4. Feste pharmazeutische orale Dosierungsform nach einem der Ansprüche 1 bis 3, wobei die Kernschicht mindestens eine Ethylcellulose umfasst.

5. Feste pharmazeutische orale Dosierungsform nach einem der Ansprüche 1 bis 4, wobei Polycaprolacton in einer Menge zwischen 20 und 99,9 Gew.-% der Kernschicht vorhanden ist.

6. Feste pharmazeutische orale Dosierungsform nach einem der Ansprüche 1 bis 3 und 5, wobei Ethylcellulose in einer Menge zwischen 20 und 99,9 Gew.-% der Kernschicht vorhanden ist.

7. Feste pharmazeutische orale Dosierungsform nach einem der Ansprüche 1 bis 6, wobei das (Co-)Polymer der Überzugsschicht in einer Menge zwischen 10 und 99,9 Gew.-% der Überzugsschicht vorhanden ist.

8. Feste pharmazeutische orale Dosierungsform nach einem der Ansprüche 1 bis 7, wobei die Kernschicht einen Durchmesser im Bereich von 0,1 bis 5 mm aufweist, und wobei die Überzugsschicht(en) eine Gesamtdicke im Bereich von 0,1 bis 3 mm aufweist (aufweisen).

9. Feste pharmazeutische orale Dosierungsform nach einem der Ansprüche 1 bis 8, wobei die Kernschicht und/oder die Überzugsschicht(en) ferner mindestens einen Weichmacher umfassen.

10. Feste pharmazeutische orale Dosierungsform nach einem der Ansprüche 1 bis 9, wobei mindestens zwei der Schichten der mehrschichtigen Freisetzungsformulierung einen aktiven Bestandteil umfassen.

11. Feste pharmazeutische orale Dosierungsform nach einem der Ansprüche 1 bis 10, wobei jede der Schichten einen aktiven Bestandteil umfasst.

12. Feste pharmazeutische orale Dosierungsform nach einem der Ansprüche 1 bis 11, wobei die Kern- und/oder die Überzugsschicht(en) denselben oder einen anderen aktiven Bestandteil umfassen.

13. Feste pharmazeutische orale Dosierungsform nach einem der Ansprüche 1 bis 12, wobei die Dosierungsform eine zylindrische Form aufweist.

14. Feste pharmazeutische orale Dosierungsform nach einem der Ansprüche 1 bis 13, zur Verwendung als Medikament.

15. Feste pharmazeutische orale Dosierungsform nach einem der Ansprüche 1 bis 13, zur Verwendung bei der Behandlung einer Krankheit durch die Abgabe eines oder mehrerer aktiver Bestandteile an ein Subjekt in einer zweiphasigen oder mehrphasigen Weise.

16. Verfahren zur Herstellung einer mehrschichtigen Freisetzungsformulierung, wobei das Verfahren umfasst: Co-Extrudieren
- einer Kernschicht, umfassend mindestens ein Polymer, ausgewählt aus Polycaprolacton, Ethylcellulose oder Kombinationen davon, und, wenn das Polymer Ethylcellulose ist, enthält die Kernschicht mindestens einen Weichmacher; und
- eine Überzugsschicht, umfassend mindestens ein (Co-)Polymer, ausgewählt aus der Gruppe bestehend aus einer Kombination von Polyethylenoxid und Polyethylenglykol; basischem butyliertem Methacrylat-(Co-)Polymer; einem (Co-)Polymer von Polyvinylcaprolactam, PEG und Polyvinylacetat; und Kombinationen davon.

17. Verfahren nach Anspruch 16, wobei das Co-Extrudat ferner zu einer Form gestaltet oder geformt wird, die ausgewählt wird aus der Gruppe bestehend aus: einem Film, einer Lage, Pellets, Kügelchen, Tabletten, Granulaten und Pulver.

## Revendications

1. Forme posologique orale pharmaceutique solide, comprenant une formulation à libération en multicouches formée par co-extrusion, ayant :
- une couche centrale, comprenant au moins un polymère choisi parmi une poly-caprolactone, l'éthyl-cellulose ou des combinaisons de celles-ci et, quand le polymère est l'éthyl-cellulose, la couche centrale contient au moins un plastifiant ; et
- une couche d'enrobage, comprenant :
• une combinaison d'oxyde de polyéthylène et de polyéthylène glycol ;
• un (co)polymère de méthacrylate butylé basique
• un (co)polymère de caprolactame polyvinylique, PEG et acétate de polyvinyle ; ou
• des combinaisons de ceux-ci ; et
dans laquelle au moins l'une desdites couches comprend un ingrédient actif.

2. Forme posologique orale pharmaceutique solide selon la revendication 1, dans laquelle la formulation à libération en multicouches comprend en outre une ou plusieurs autre(s) couche(s) centrale(s) et/ou d'enrobage.

3. Forme posologique orale pharmaceutique solide selon les revendications 1 ou 2, dans laquelle la couche centrale comprend au moins une poly-caprolactone.

4. Forme posologique orale pharmaceutique solide selon l'une quelconque des revendications 1 à 3, dans laquelle la couche centrale comprend au moins une éthyl-cellulose.

5. Forme posologique orale pharmaceutique solide selon l'une quelconque des revendications 1 à 4, dans laquelle la poly-caprolactone est présente en une quantité comprise entre 20 et 99,9 % en poids de la couche centrale.

6. Forme posologique orale pharmaceutique solide selon l'une quelconque des revendications 1 à 3 et 5, dans laquelle l'éthyl-cellulose est présente en une quantité comprise entre 20 et 99,9 % en poids de la couche centrale.

7. Forme posologique orale pharmaceutique solide selon l'une quelconque des revendications 1 à 6, dans laquelle le (co)polymère de la couche d'enrobage est présent en une quantité comprise entre 10 et 99,9 % en poids de la couche d'enrobage.

8. Forme posologique orale pharmaceutique solide selon l'une quelconque des revendications 1 à 7, dans laquelle la couche centrale a un diamètre compris dans la gamme des 0,1 à 5 mm et dans laquelle la/les couche(s) d'enrobage a/ont une épaisseur totale comprise dans la gamme des 0,1 à 3 mm.

9. Forme posologique orale pharmaceutique solide selon l'une quelconque des revendications 1 à 8, dans laquelle la couche centrale et/ou la/les couche(s) d'enrobage comprennent en outre au moins un plastifiant.

10. Forme posologique orale pharmaceutique solide selon l'une quelconque des revendications 1 à 9, dans laquelle au moins deux des couches de la formulation à libération en multicouches comprennent un ingrédient actif.

11. Forme posologique orale pharmaceutique solide selon l'une quelconque des revendications 1 à 10, dans laquelle chacune des couches comprend un ingrédient actif.

12. Forme posologique orale pharmaceutique solide selon l'une quelconque des revendications 1 à 11, dans laquelle les couches centrale et d'enrobage comprennent le même ingrédient actif ou un différent.

13. Forme posologique orale pharmaceutique solide selon l'une quelconque des revendications 1 à 12, dans laquelle la forme posologique a une forme cylindrique.

14. Forme posologique orale pharmaceutique solide, selon l'une quelconque des revendications 1 à 13, destinée à être utilisée comme médicament.

15. Forme posologique orale pharmaceutique solide, selon l'une quelconque des revendications 1 à 13, destinée à être utilisée dans le traitement d'une maladie par la délivrance d'un ou de plusieurs ingrédient(s) actif(s) à un sujet d'une manière biphasée ou multiphasée.

16. Procédé de préparation d'une formulation à libération en multicouches ; ledit procédé comprenant la co-extrusion de :
- une couche centrale, comprenant au moins un polymère choisi parmi une poly-caprolactone, l'éthyl-cellulose ou des combinaisons de celles-ci et, quand le polymère est l'éthyl-cellulose, la couche centrale comprend au moins un plastifiant ; et
- une couche d'enrobage, comprenant au moins un (co)polymère choisi dans le groupe constitué par une combinaison d'oxyde de polyéthylène et de polyéthylène glycol ; d'un (co)polymère de méthacrylate butylé basique ; d'un (co)polymère de caprolactame polyvinylique, PEG et acétate de polyvinyle ; et des combinaisons de ceux-ci.

17. Procédé selon la revendication 16, dans lequel le coextrudat est façonné ou moulé en outre en une forme choisie dans le groupe constitué par : un film, une feuille, des pilules, des billes, des comprimés, des granules et une poudre.
